**Europäisches Patentamt**

(19) **European Patent Office**

**Office européen des brevets**

(11) Publication number: **0 139 051**
**B1**

(12) # EUROPEAN PATENT SPECIFICATION

(45) Date of publication of patent specification: **25.11.87**

(51) Int. Cl.⁴: **B 01 J 35/10,** B 01 J 23/72, C 07 C 7/163

(21) Application number: **83306347.2**

(22) Date of filing: **19.10.83**

(54) Catalyst for selective hydrogenation of alkynes in the presence of dienes.

(43) Date of publication of application:
**02.05.85 Bulletin 85/18**

(45) Publication of the grant of the patent:
**25.11.87 Bulletin 87/48**

(84) Designated Contracting States:
**BE DE FR GB IT NL**

(56) References cited:
DE-A-2 828 034
GB-A- 945 403
GB-A-1 422 451
GB-A-1 422 452
GB-A-1 465 523
GB-A-2 073 162
US-A-3 692 698
US-A-3 912 789
US-A-4 101 451

**Bulletin CC-1 catalyst Corriers'Norton**

The file contains technical information submitted after the application was filed and not included in this specification

(73) Proprietor: **THE DOW CHEMICAL COMPANY**
**2030 Dow Center Abbott Road P.O. Box 1967**
**Midland, MI 48640 (US)**

(72) Inventor: **Couvillion, Mark Campbell**
**1314 W. 7th**
**Freeport Texas 77541 (US)**

(74) Representative: **Burford, Anthony Frederick**
**et al**
**W.H. Beck, Greener & Co. 7 Stone Buildings**
**Lincoln's Inn**
**London WC2A 3SZ (GB)**

Courier Press, Leamington Spa, England.

**Description**

This invention relates to catalysts useful for the hydrogenation of alkynes e.g. to remove such alkynes from a hydrocarbon stream, and to methods for preparing such catalysts.

Copper metal, activated with one or more of the metals silver, chromium and/or molybdenum on an alumina support, is well known as a catalyst for hydrogenation of alkynes. Frevel et al. have issued several Patents in which improved results are obtained (improved selectivity of hydrogenation of carbon-carbon triple bonds in the presence of diene bonds) by increasing the sodium content of the activated catalysts, U.S. Patent 4,101,451, and/or high sodium oxide alumina, U.S. Patent 3,912,789. However, these catalysts still hydrogenate some considerable amount of the diene components of such streams. As the diene content of these streams becomes more valuable it would be advantageous to reduce the hydrogenation of these dienes (particularly 1,3 butadiene) without also reducing the hydrogenation of the alkynes.

It has now been found that by employing a gamma alumina, (preferably containing not more than 50 percent by weight, more preferably not more than 35 percent by weight of alpha alumina), having a surface area of between 68 and 350 square meters per gram, 90 to 50 percent (preferably 90 to 55, more preferably 90 to 60 percent) of the pore volume being in pores individually having an average pore diameter between 4 nm and 12 nm and not more than 25 percent of the pore volume nor less than 0.5 per cent (preferably not less than 1 percent, more preferably not less than 2 percent) being in pores individually having an average pore diameter between 100 nm to 1,000 nm, produces a catalyst support which when coated with copper and optionally an activator metal, permits reaching lower alkyne levels with less alkene loss than prior art catalysts, and additionally can be effectively regenerated to 90 plus percent activity over several cycles. The gamma alumina preferably has one or more of the following characteristics: less than 0.15 weight percent silica as $SiO_2$; less than 0.15 weight percent, more preferably less than 0.10 weight percent sodium as $Na_2O$; less than 0.06 weight percent iron as $Fe_2O_3$; and less than 0.01 weight percent sulfur.

Preferably the catalyst includes at least one activator metal such as silver, platinum, palladium, manganese, cobalt, nickel, chromium or molybdenum. The activator metal may be present at up to 15% by weight of the copper. The copper preferably is present at a level of 3 to 13% of the weight of the support, particularly if there is an activator metal present also at the level of up to 15% by weight of the copper.

The invention includes processes for the hydrogenation of alkynes, e.g. the selective hydrogenation of alkynes in a hydrocarbon stream containing a diene such as 1,3-butadiene, comprising contacting the alkynes with such a catalyst under appropriate reaction conditions.

It has also been found that during use the gamma alumina described above undergoes a phase change, probably due to thermal treatment during operation and regeneration. Thus, a gamma alumina within the scope above defined and essentially free of alpha alumina will undergo a gradual change, as for example, during a nine month, thirteen cycle (with regeneration) run, to analyze about 35 percent alpha alumina with an attendant reduction in surface area from about 165 square meters per gram to about 68 square meters per gram. There is also a change in the pore volume distribution associated with the reduction in surface area and presence of the alpha alumina. However, the pore sizes remain within the aforedefined range. Thus, while it is preferred to start with a relatively high purity gamma alumina having the above described properties and physical characteristics, it is to be understood that a carrier or support may be a combined unitary alumina consisting of a high purity alumina as above defined but having a content of alpha alumina, preferably not exceeding fifty percent alpha alumina in admixture with gamma alumina. Such a support can be readily obtained by thermal treatment of gamma alumina of the requisite purity in the presence of cuprous oxide. The phase change apparently occurs during the oxidation and/or reduction during regeneration. Presumably the cuprous oxide acts as a seed to effectuate the phase change from gamma to alpha state. However, no specific theory based on scientific data of how such a change occurs can yet be set forth. While the aforestated procedure appears to effect the change, other methods may be available from those skilled in the art of alumina production.

The catalyst carrier or support which appears to be greatly preferred for obtaining the aforesaid results is a special grade of gamma alumina ($\gamma$-$AL_2O_3$) prepared by decomposing trialkyl alumina to alpha aluminum monohydrate then calcining the alpha aluminum monohydrate to gamma alumina. This process produces a grade of gamma alumina normally not obtainable from naturally occurring aluminum containing ores and which has, when pressed or extruded into pellets, a higher purity than naturally occurring alumina, and a pore size and pore size distribution sufficiently different from that obtainable using naturally occurring aluminas and converting them to gamma alumina. Throughout this specification, by the term "gamma alumina" is meant a true gamma alumina. Such a true gamma alumina will match the published X-ray diffraction pattern (# 29—63) of the Joint Committee on Powder Diffraction Standards (a non-profit scientific body based in Philadelphia, PA, USA). The most ready sources of catalyst support size pellets are Norton Company, sold as SA6173 and Calcicat Division, Mallinckrodt Chemical Works as CALCICAT Type A and AA. Although Conoco Chemicals Division, Conoco, Inc., manufactures a powder, CATAPAL type SB which has been found suitable and is believed to be the precursor powder for the two pellet producers, suitable pellets produced by Conoco are not readily available except through the two pelletizers. Moreover a suitable carrier material is disclosed in GB—A—14 22 452.

The alkynes present in a typical feed stream will usually be a mixture of $C_2$ to $C_6$ alkynes, and more commonly $C_3$—$C_4$ alkynes. A typical feed stream will have 1-butyne-3-ene (vinyl acetylene) as the major

0 139 051

alkyne component, along with lesser quantities of 1-butyne (ethyl acetylene) and 1-propyne (methyl acetylene).

While the catalysts of the invention may be used to hydrogenate virtually any alkyne feed stream, a liquid stream is preferred. A particular advantage of these catalysts is their selectivity for carbon-carbon triple bonds to the relative exclusion of carbon-carbon double bonds. Thus, these catalysts have extreme economic importance for the removal of alkynes from alkenes. A typical situation in which these catalysts may be used is the removal of mixed alkynes from a mixture of 1,3-butadiene and 1-butene. These catalysts will cause very little hydrogenation of the 1,3-butadiene into the much less valuable 1-butene. In fact, if 1-butyne-3-ene is the major alkyne contaminant, it is possible to actually increase the level of 1,3-butadiene by the hydrogenation of the alkyne contaminant.

The drawings, Figures 1—9 and 16—20, illustrate the results of the several experiments employing different supports as carriers for the catalytically active components, with respect to the alkynes in the product stream and the loss of 1,3-butadiene from the feed stream as a result of the process. Figures 10, 11, 14 and 15 illustrate the pore size distribution of the several supports of the examples, and Figures 12 and 13 illustrate the results obtained when the heretofore conventional support, γ-AlOOH, REYNOLDS RA—1 is employed. Figure 21 illustrates the effect of water vapor on copper crystallite size and, in conjunction with Example 8, the results of such growth on catalyst activity.

Example 1

In accordance with the present invention 300 grams of $\gamma\text{-Al}_2\text{O}_3$ obtained from Conoco Chemicals Division, Conoco, Inc., as ⅛ inch (3.2 mm) diameter by ¼ inch (6.3 mm) long pellets identified as CATAPAL SB had the following properties:

x-ray diffraction pattern machines Joint Committee on Powder Diffraction standards (# 29—63);

| | |
|---|---|
| % $Na_2O$ | 0.004 |
| Sulfur | 0.01 |
| % $SiO_2$ | 0.008 |
| % $Fe_2O_3$ | 0.005 |
| Surface area (m²/g) | 241 |
| Pore volume (cc/g) | 0.57 |
| Bulk density (g/cc) | 0.79 |

75% of its pore volume was in pores less than 7.5 nm average diameter
82% of its pore volume was in pores less than 10 nm average diameter
18% of the pore volume was in pores between 10.2 nm and 839 nm average diameter
The unburdened support was impregnated with a solution consisting of:

| | | |
|---|---|---|
| $Cu(NO_3)_2.2\frac{1}{2}H_2O$ | 113 | g |
| $Ni(NO_3)_2.6H_2O$ | 4.0 | g |
| $H_2O$ | ~40 | g |
| $HNO_3$ | 59 | g |
| $Mn(NO_3)_2$ 50% active | 3.1 | g |
| $AgNO_3$ | 0.2 | g |
| $Cr(NO_3)_3.9H_2O$ | 1.7 | g |
| $Co(NO_3)_2.6H_2O$ | 1.7 | g |

Solubilization of the numerous metal salts was obtained by gentle heating. The resulting solution was poured over 300 grams of the support in a beaker while stirring to obtain even distribution. When all of the solution had been sorbed, the support was dried over night at 110°C, then calcined at 400°C for about 6 hours.

The catalyst was loaded to a depth of about 12 inches (305 mm) at the middle of a laboratory 1 inch (25.4 mm) diameter by 36 inch (914 mm) long reactor. The remaining space of the reactor, above and below

3

the catalyst, was filled with raschig rings. The catalyst was reduced over night with hydrogen at between 300°C to 350°C. In the morning the temperature recording from several thermocouples in the bed showed an exotherm had passed up the column during the night signifying reduction of the metal oxides to their metal state. The reactor was cooled to ambient temperature and hydrogen and a liquid hydrocarbon stream having about 61% by weight 1,3-butadiene and 8716 ppm mixed alkynes was fed to the reactor. The reactor conditions were:

> Feed 300 cc/hour
> Recycle 600 cc/hour
> $H_2$ Flow 2.8 liters/hr at atm. STP
> $H_2$: C≡ ratio 4:1
> Inlet temperature of liquid 60°C
> T at thermocouple #1 ca. 70°C
> T at thermocouple #2 ca. 67°C
> T at thermocouple #3 ca. 66°C
> T at thermocouple #4 ca. 62°C
> T at thermocouple #5 ca. 58°C

Regeneration was accomplished by purging the reactor with $N_2$ at about 12 liters/min., followed by introduction of air first at 18.15 liters/min. then 13.3 liters/min. Finally the air was shut off and $H_2$ was admitted at 4.1 liters/min. The GHSV (gas hourly space velocity values) were: $N_2$ 1650 $hr^{-1}$, $N_2$ + air (max.) 3460 $hr^{-1}$; and, $N_2$ + $H_2$ 2215 $hr^{-1}$, respectively.

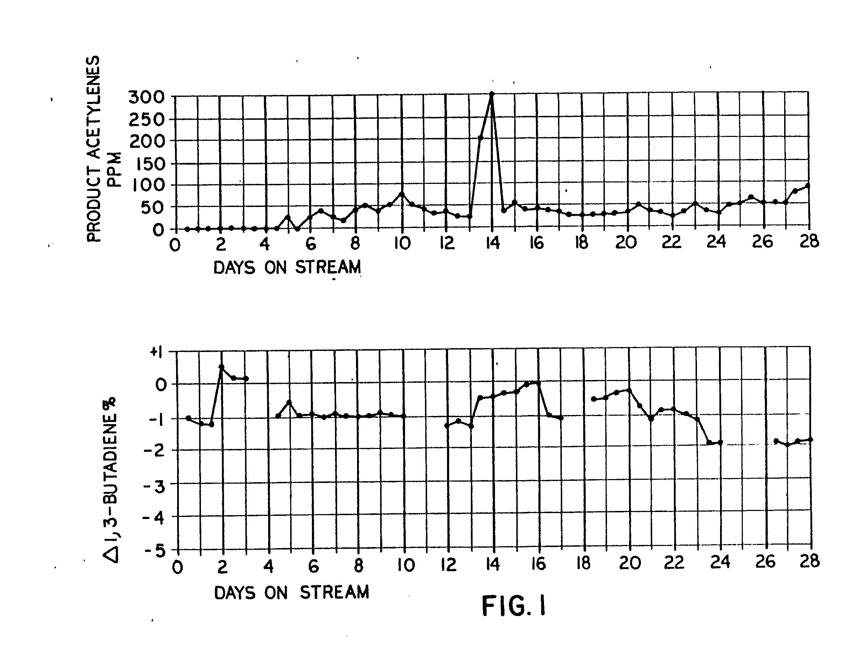

The results of two runs with regeneration are illustrated in Figure 1 which shows that the product had an average of less than 80 ppm alkynes having hydrogenated over 8630 ppm of the alkynes in the feed with less than 1% total butadiene loss based on the butadiene present in the feed.

## Example 2

To 400 grams of a catalyst support obtained from Calsicat Division, Mallinckrodt Chemical Works, identified as CALSICAT Type A having the following physical properties as $\frac{1}{8}$ inch (3.2 mm) pellets:

> x-ray diffraction pattern matches Joint Committee
> on Powder Diffraction Standards #29—63;

| | |
|---|---|
| % $Na_2O$ | 0.005 |
| % $SiO_2$ | 0.01 |
| % $Fe_2O_3$ | (<0.1) |
| % Sulfur | 0.01 |
| Surface area ($m^2$/g) | 200 |
| Pore volume (cc/g) | 0.50 |
| Bulk density (g/cc) | 0.8 |

was added the following liquid mixture:

| | grams |
|---|---|
| $Cu(NO_3)_2 \cdot 2\frac{1}{2}H_2O$ | 140 |
| $Ni(NO_3) \cdot 6H_2O$ | 4.75 |
| $Mn(NO_3)_2$ 50% soluiton | 4 |
| $Co(NO_3)_2 \cdot 6H_2O$ | 2 |
| $Cr(NO_3)_3 \cdot 9H_2O$ | 2 |
| $HNO_3$ (conc.) | 7.1 |
| $AgNO_3$ | 0.25 |
| $H_2O$ | ~50 |

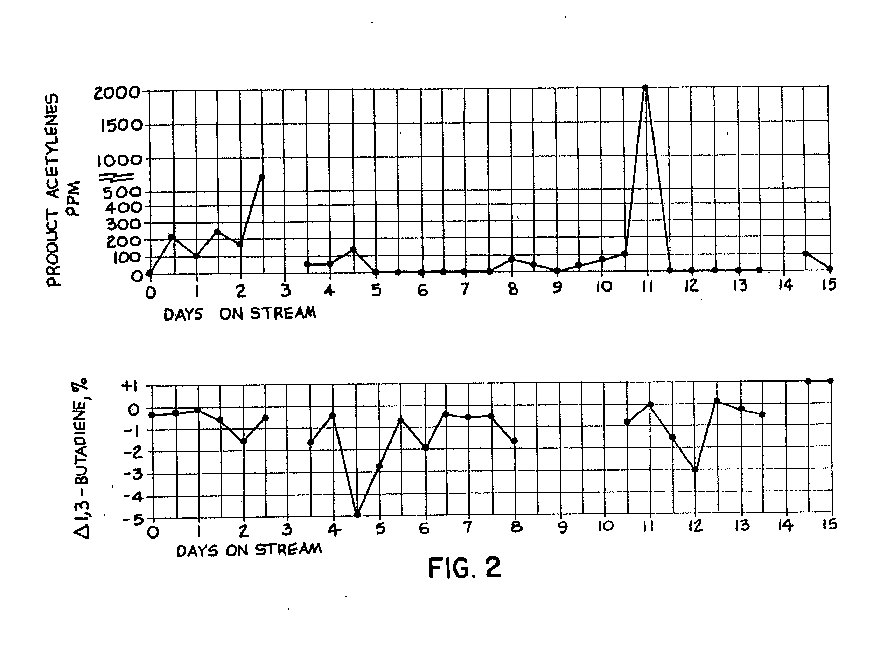

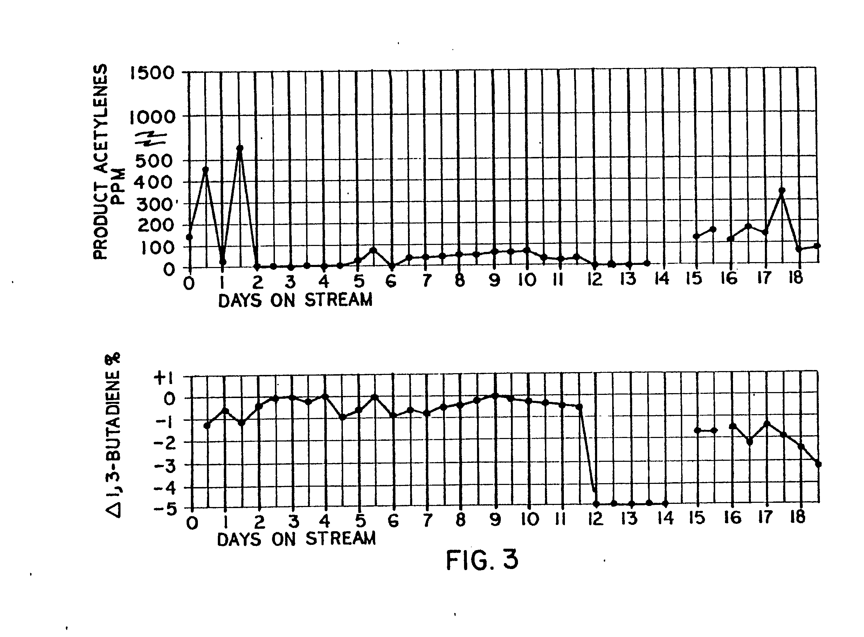

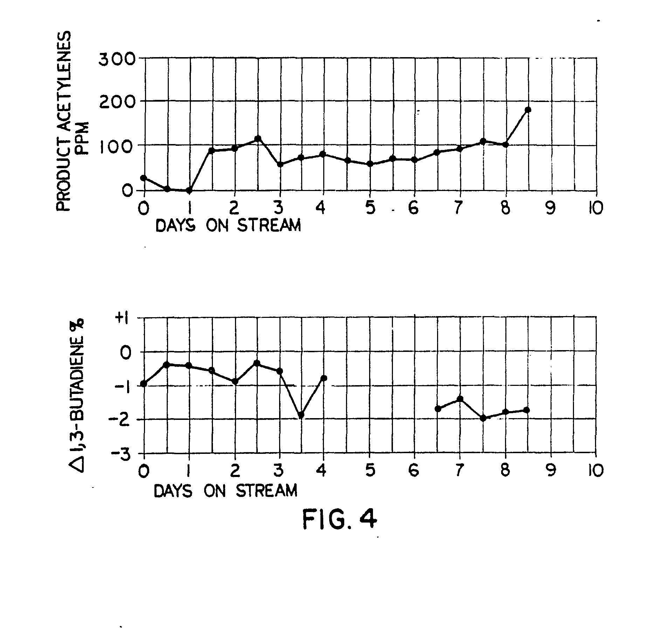

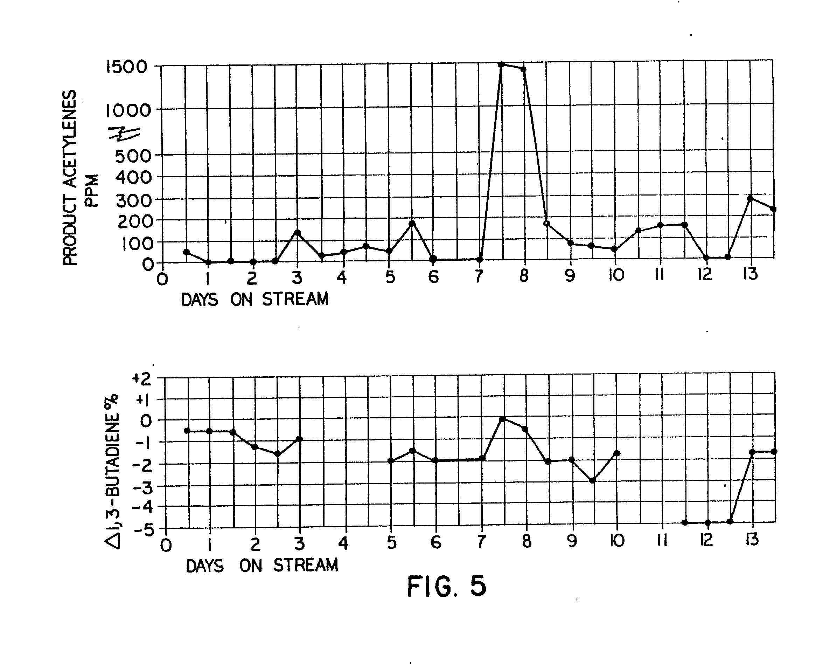

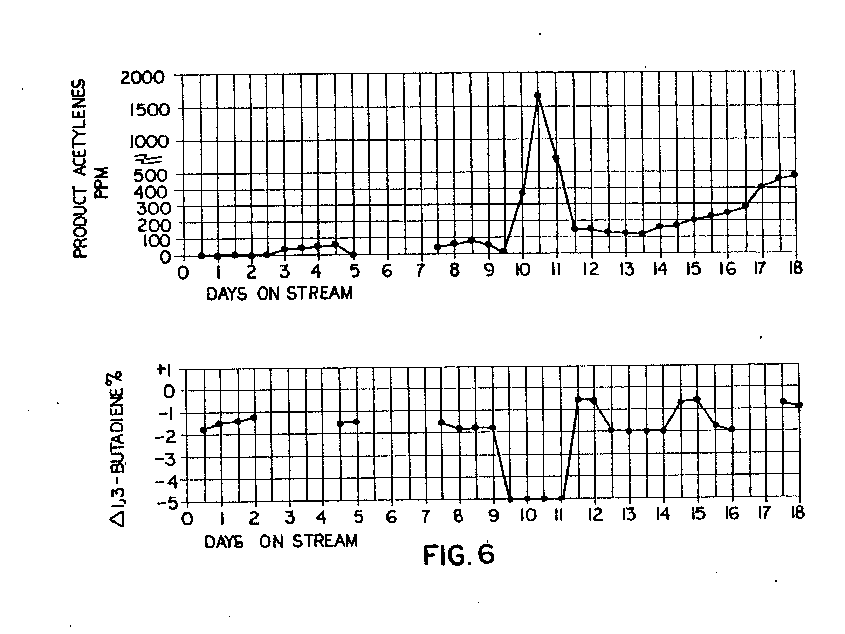

with stirring and heating, 50—60°C, until the liquid was sorbed. Thereafter the wetting support was dried in an oven at 110°C for 2 hours, then placed in a furnace, cooled and packed in a reactor of like size and in the same manner as described in Example 1. The 72-day results of 5 cycles with regeneration as previously described are illustrated in Figures 2—6. The average 1-alkyne content of the outlet product was less than 100 ppm (from 8000—10,000 ppm in feed) and a loss of butadiene of less than 1 percent of that in the feed.

Example 3

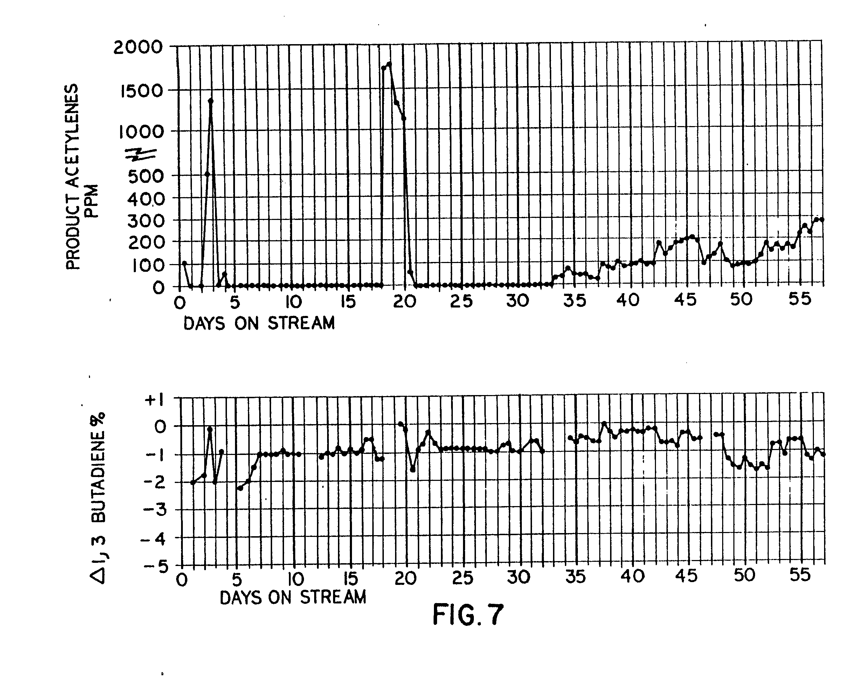

In a similar manner as Example 2, 300 grams of a 1/8 inch (3.2 mm) pellet CALSICAT type AA catalyst support was impregnated with a proportionate volume of the catalyst formulation, dried, packed in a similar reactor, reduced with $H_2$, and operated without regeneration for 57 days. The results of a two-month cycle, illustrated in Figure 7, show an average of <50 ppm alkyne coming through during the first 35 days and < 150 pm alkyne coming through the next 22 days. The loss of butadiene during the entire 57 days was about 1 percent.

Comparative Example 1

Likewise, a catalyst prepared on HARSHAW A1—3438T support having porosity characteristics not in accordance with the invention, was prepared and tested in a similar manner to the foregoing Examples. The results of six (6) days operation showed 200 ppm alkyne (avg.) remaining in the treated liquids and a loss of about 2 percent butadiene.

Example 4

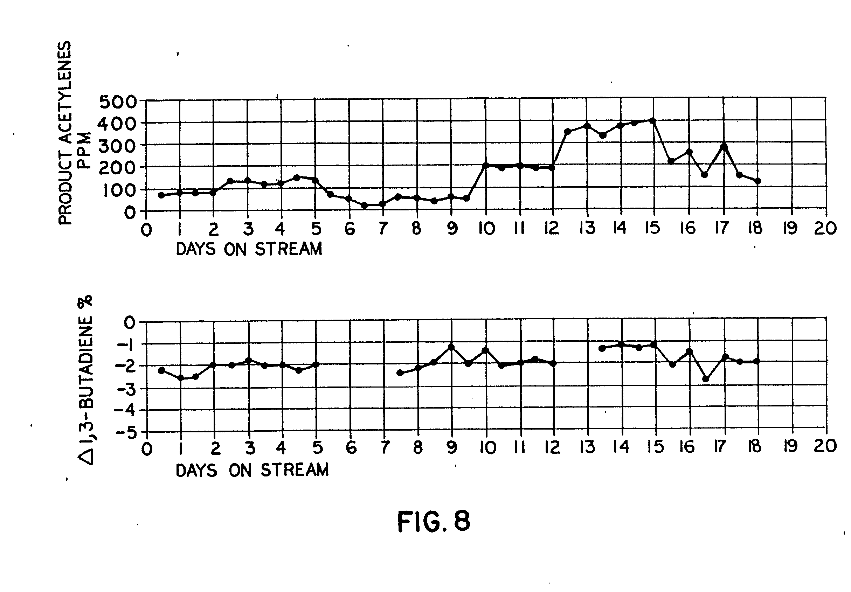

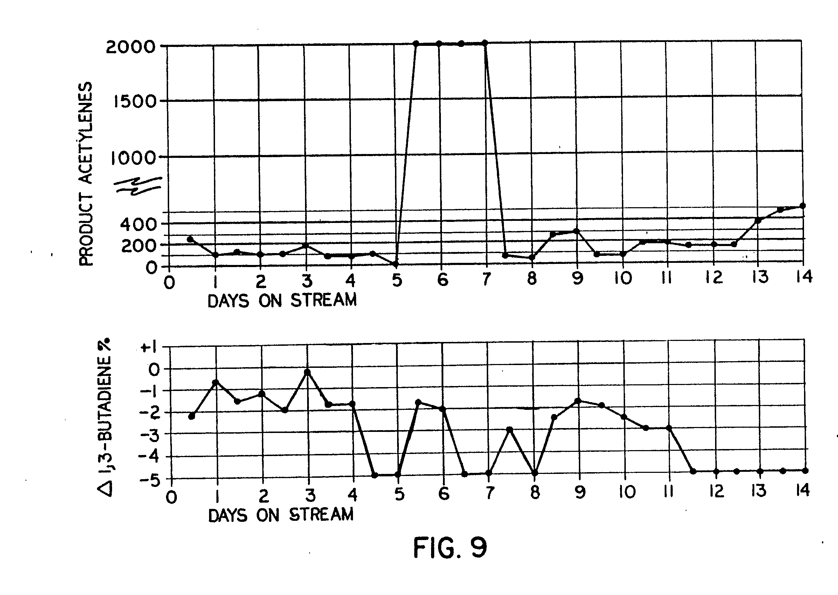

A NORTON SA6173 support was employed to prepare a catalyst and operated for a first cycle of 18 days in a test as afore described. Regeneration occurred on the 18th day. The processed liquids had an average of 200 ppm alkyne and about a 1 percent loss in butadiene content on the initial run. The butadiene losses increased from 2 to 4 percent on the regenerated catalyst over a 15-day second cycle. The graphic daily results are shown in Figure 8 and 9.

In order to identify each support, the physical properties of each are set forth in Table I.

TABLE I

| | % Na$_2$O | SiO$_2$ % | % Fe$_2$O$_3$ | Surface Area m$^2$/g | Pore Volume cc/g | Bulk Density g/cc | Sulfur ([2]) |
|---|---|---|---|---|---|---|---|
| CALSICAT Type A | 0.005 | 0.01 | (<0.01) | 200 | 0.50 | 0.80 | (0.01) |
| HARSHAW A1—3438T | (0.005) | (0.01) | <0.01 | 175 | 0.50 | 0.78 | (0.01) |
| CATAPAL Type SB | 0.004 | 0.008 | 0.005 | 241 | 0.57 | 0.79 | 0.01 |
| CALSICAT Type AA | 0.07 | 0.12 | <0.06 | 215 | 0.70 | 0.63 | — |
| NORTON SA—6173 | 0.015 | 0.09 | 0.06 | 240 | 0.56 | 0.69 | — |
| REYNOLDS RA—1([1]) | 0.35 | 66 ppm | 190 ppm | 213 | 0.18 | 0.88 | — |

[1] Conventional γ-AlOOH support.
[2] No value listed indicates the level present was below detection limits.
Note: values in parentheses are assumed values.

# 0 139 051

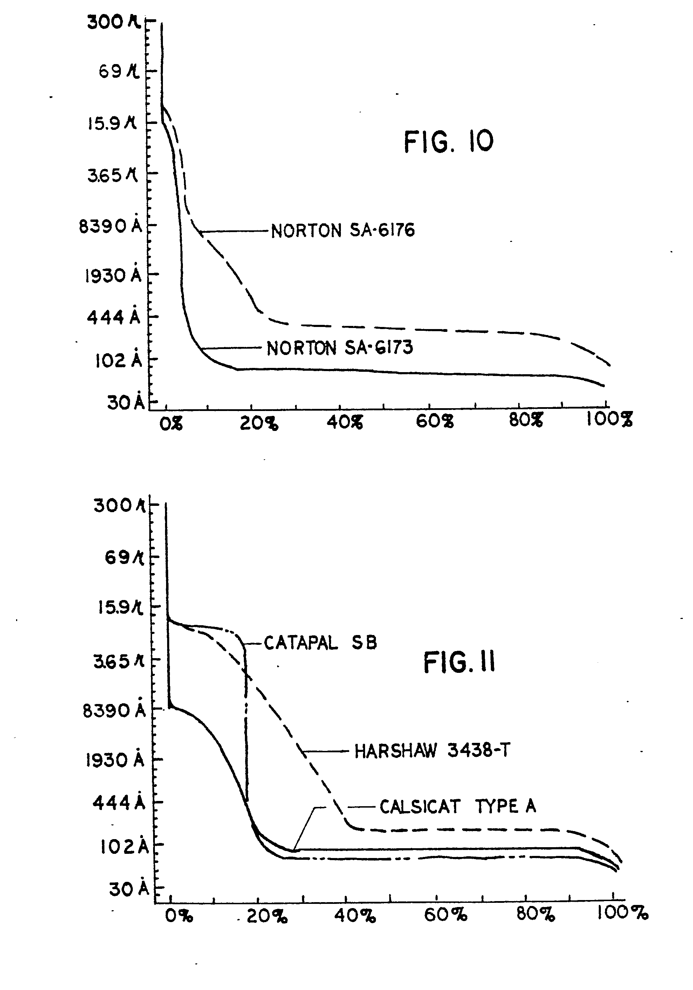

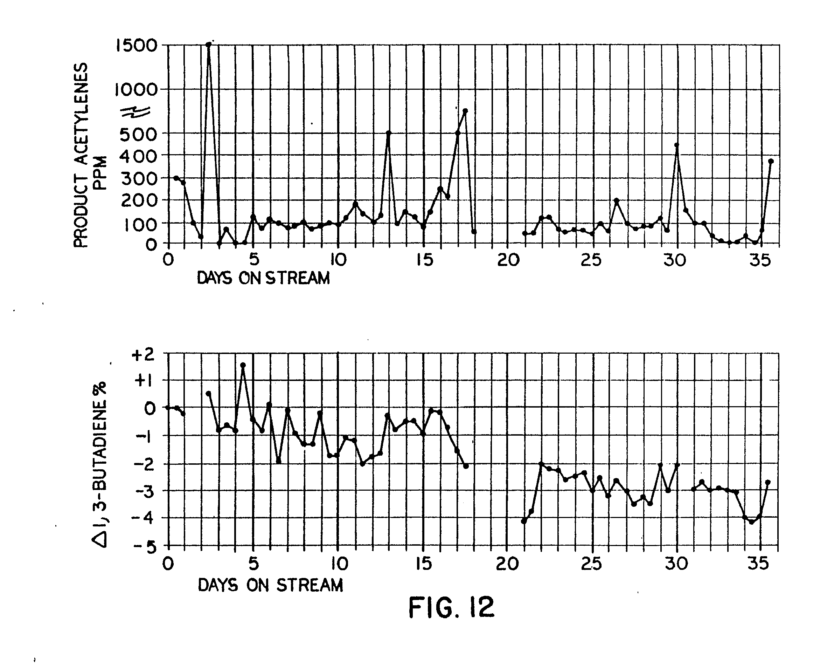

The pore distribution of each support is shown in the drawings, Figures 10 and 11. In Figures 10, 11, 14 and 15 "Å" refers to $10^{-10}$ meters, and $\mu$ refers to $\mu m$.

## Comparative Example 2

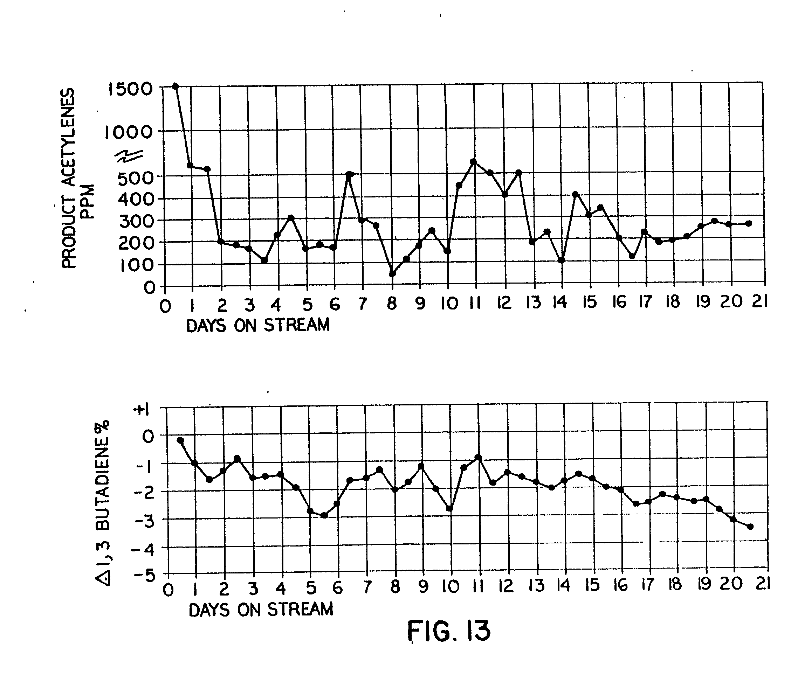

For purposes of comparison, a catalyst having the exact composition as that applied in Example 1 was applied to a conventional gamma alumina (REYNOLDS RA—1 a $\gamma$-AlOOH) having a normal sodium content and pore size distribution. The results of 2 cycles, with regeneration after the 35th day of the first cycle, shows results illustrated in Figures 12 and 13; to wit: 100—200 ppm of mixed alkynes during the first cycle rising to 200—400 during the second cycle, with loss of 1,3-butadiene in the 1 to 4 percent range in both cycles.

## Example 5

A catalyst prepared from a high purity Norton SA—6173 1/16 inch (1.59 mm) extrudates upon which copper and promoter metals were impregnated, was calcined at 400°C for 8 hours. The proportions employed to prepare this catalyst were:

|  | grams |
|---|---|
| Norton SA—6173 1/16 inch | 4800 |
| $Cu(NO_3)_2 \cdot 2\frac{1}{2}H_2O$ | 1680 |
| $Ni(NO_3) \cdot 6H_2O$ | 57 |
| $Mn(NO_3)_2$ 50% solution | 48 |
| $Co(NO_3)_2 \cdot 6H_2O$ | 24 |
| $Cr(NO_3)_3 \cdot 9H_2O$ | 24 |
| $HNO_3$ (conc.) | 85 |
| $AgNO_3$ | 3 |
| $H_2O$ | ~600 |

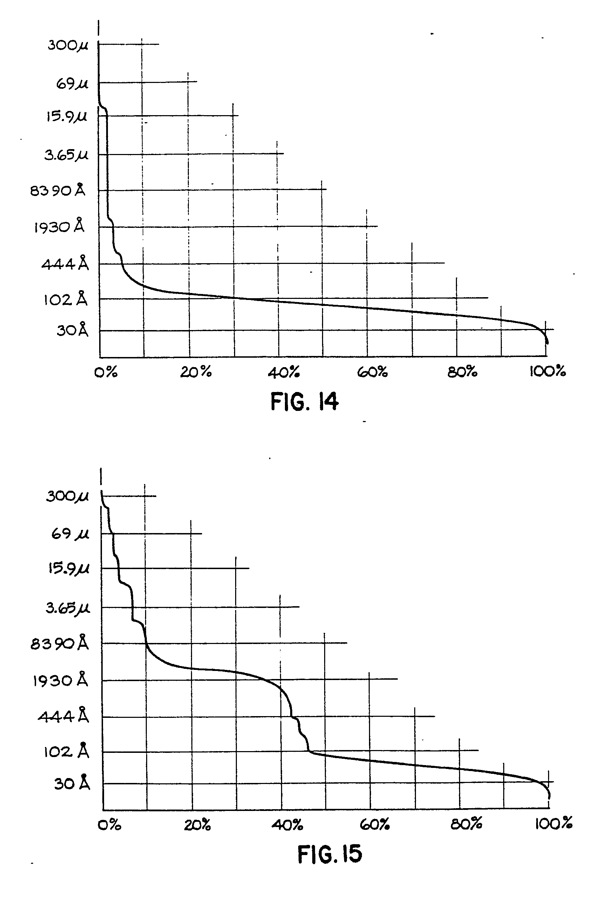

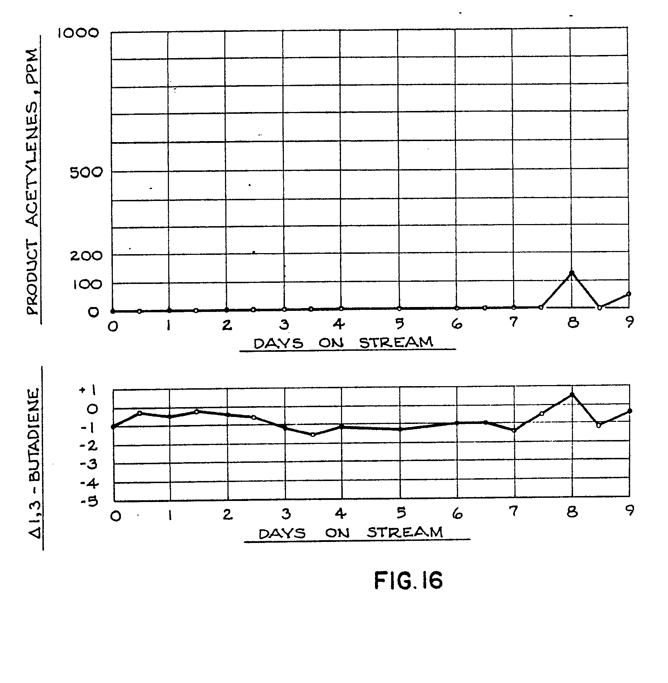

The calcined catalyst was loaded into a reactor, heated to 250°C in the presence of nitrogen then reduced with up to 5 volume percent $H_2$ in nitrogen for about 12 hours. The reactor was cooled and liquid hydrocarbon was introduced into the reactor and catalyst bed. The reactor temperature was maintained between about 50°C and 70°C. Hydrogen to alkyne molar ratio was maintained at about 3 to 4 throughout thirteen cycles. Regeneration of the catalyst was carried out about every 14 to 21 days by heating to about 250°C to 300°C in the presence of nitrogen, then in the presence of air until an exotherm had passed through the bed, and then maintained on air for 2—4 hours, until no other exotherm wave was observed. The air was stopped and hydrogen of up to 5 volume percent in nitrogen passed through the bed until an exotherm moved through the bed. When no additional exotherm was observed within 2 to 4 hours on hydrogen, the bed was cooled and placed on line with hydrocarbon feed. The catalyst carrier had the initial pore volume distribution shown in Fig. 14 and a BET nitrogen surface area of 165 m²/g. Following the twelfth cycle before regeneration the carrier had a pore volume distribution as shown in Fig. 15. The BET nitrogen surface area was 68 m²/g.

The initial data of operation for the 13th cycle for 3 days was comparable to the initial 3 days operation of the first and subsequent cycles.

## Example 6

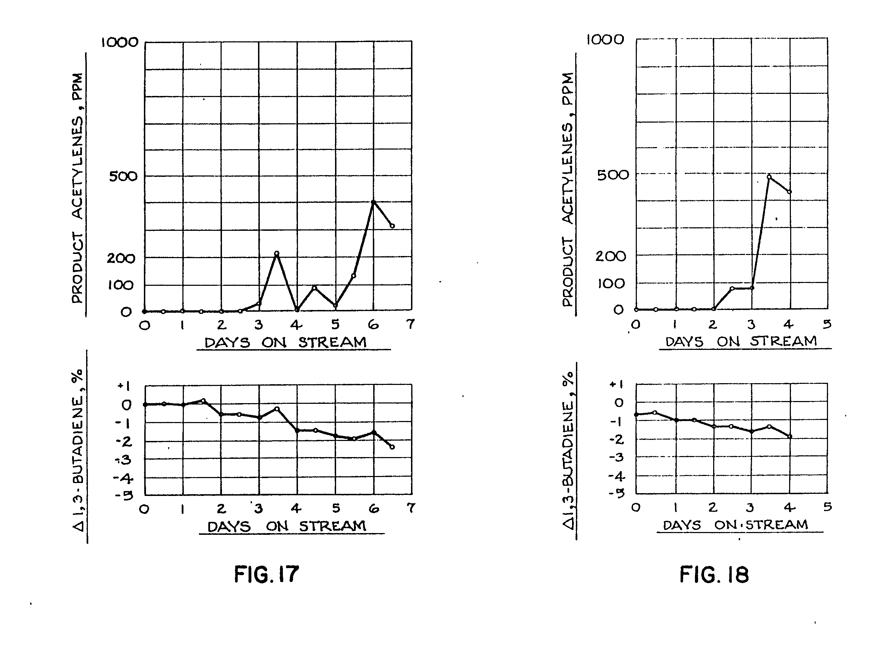

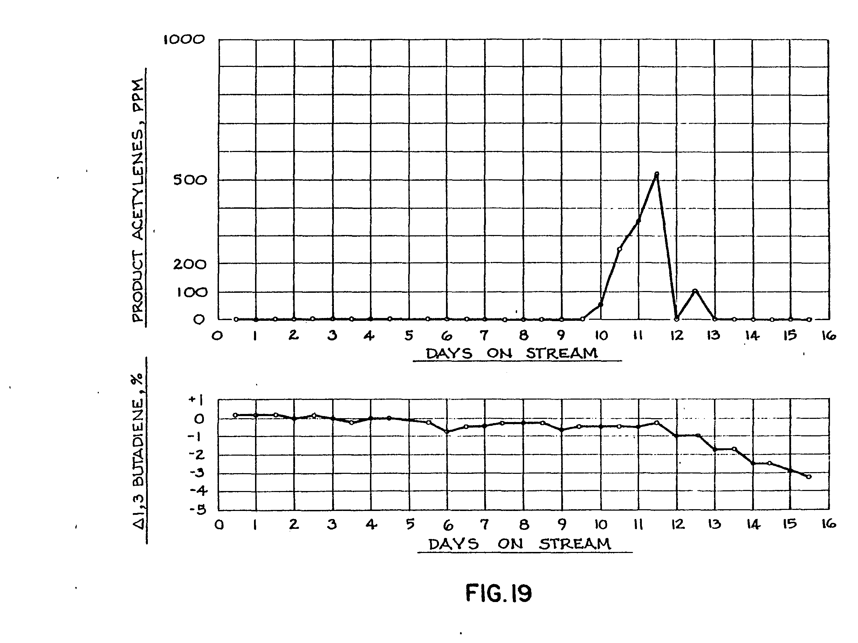

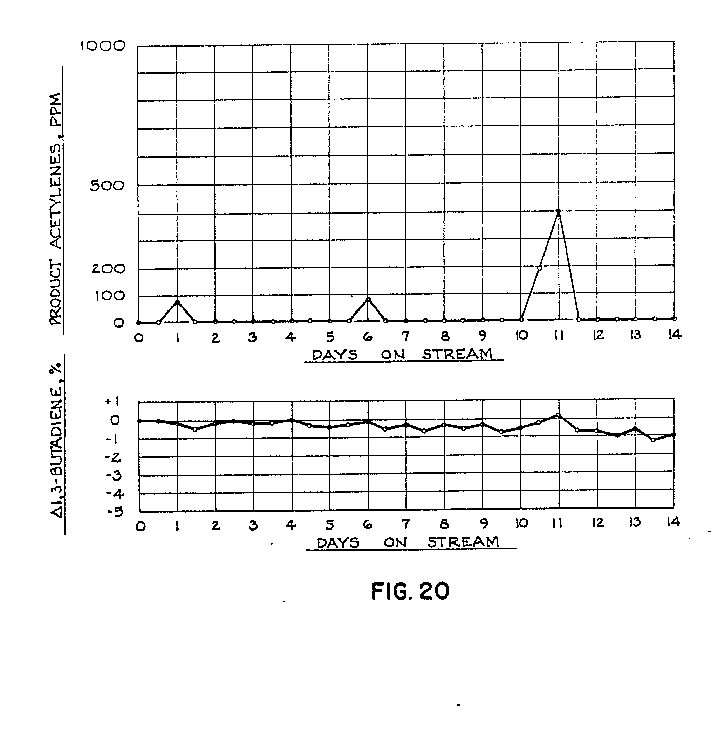

In evaluating the performance of the catalyst and its support in respect to materials of construction, it was found that the materials of construction for the reactor are critical if long, fourteen day, on stream cycles are desired. Thus when one employs a stainless steel containing nickel, the efficiencies of the catalysts of the present invention are somewhat reduced due to the necessity to regenerate the catalyst more often. This phenomenom is overcome when carbon steel is employed as the material of construction for the reactor. The effects of the presence of nickel even though only small amounts are possible at the internal surface of the reactor (wall effect) are clearly seen in Figures 16, 17, and 18, the 9th, 12th, and 13th cycles, in a 304 stainless steel reactor. The use of a carbon steel reactor illustrates the improved efficiency, i.e. longer run time between regeneration before the alkynes are no longer effectually hydrogenated and is shown by comparing results of the three just mentioned runs, Figures 16, 17, and 18, with the results of the carbon steel reactor shown in Figures 19 and 20.

7

Example 7

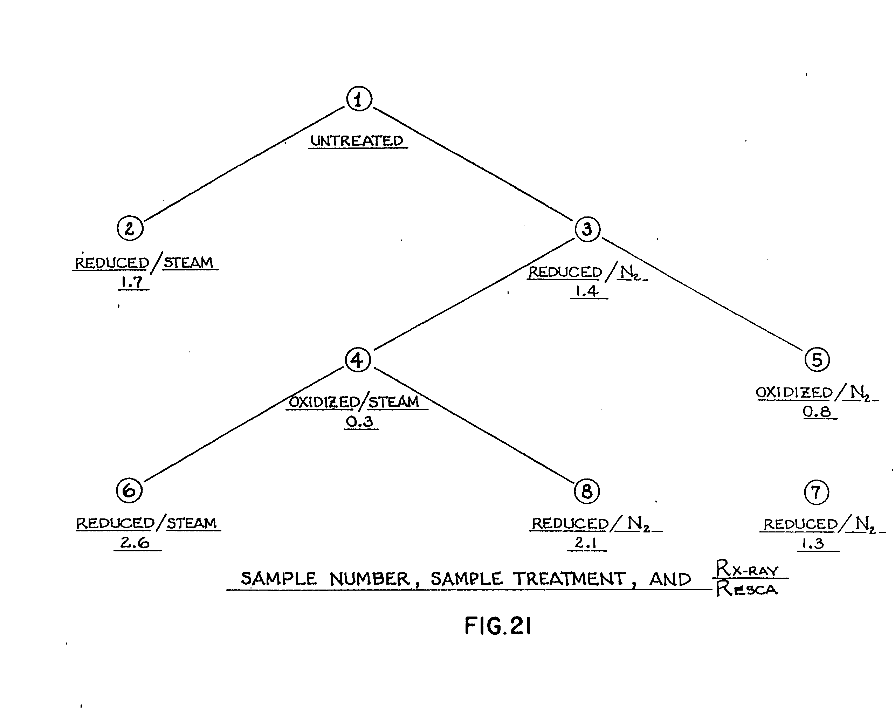

Another point which long run data establishes is that steam regeneration markedly reduces the life of a catalyst by a loss of surface copper and an increase in copper crystallite size following regeneration and/or oxidation of the catalyst. Results are illustrated in the Table II. Samples were prepared in the sequence shown in Figure 21.

## TABLE II
### COMPARISON OF STEAM AND $N_2$ DILUENTS DURING REGENERATION

| SAMPLE | DILUENT | FINAL OXIDATION STATE | % Cu++ | $Cu°/\gamma\text{-}Al_2O_3$ ESCA SMALL SURFACE SPECIES | $Cu°/\gamma\text{-}Al_2O_3$ X-RAY LARGE BULK SPECIES | R x-ray / R ESCA[2] LARGE TO SMALL CRYSTAL RATIO |
|---|---|---|---|---|---|---|
| 1 | — | Ox. | 100 | 1.58 | — | — |
| 2 | Steam | Red. | 5 | 0.50 | 0.86 | 1.7 |
| 3 | $N_2$ | Red. | 5 | 0.83 | 1.16 | 1.4 |
| 4 | Steam | Ox. | 73 | 0.97 | 0.25 | 0.3 |
| 5 | $N_2$ | Ox. | 29 | 0.89 | 0.75 | 0.8 |
| 6 | Steam | Red. | 1 | 0.27 | 0.69 | 2.6 |
| 7 | $N_2$ | Red. | 19 | 0.60 | 0.78 | 1.3 |
| 8 | $N_2$ | Red. | 7 | 0.54 | 1.13 | 2.1 |

[1] Total area for ESCA scan of the Cu 2p3/2 peak to total area per ESCA scan of the aluminum 2s peak was taken as amount of small copper particles present in the catalyst.

[2] The ratio of the height of the copper 0.209 nm peak by x-ray diffraction to the $\gamma\text{-}Al_2O_3$ 0.140 nm peak of x-ray diffraction was calculated as a measure of the large copper crystallites present. Ratio of x-ray ratio to ESCA ratio was calculated as ratio of large to small copper crystallites.

## 0 139 051

**Claims**

1. A catalyst for the hydrogenation of alkynes, comprising a support having thereon finely divided copper metal, characterised in that the support is a porous gamma alumina optionally containing alpha-alumina and having a surface area of 68 to 350 $m^2/g$, 50 to 90 percent of the pore volume being in pores individually having an average diameter of 4 to 12 nm, and 0.5 to 25 pecent of the pore volume being in pores individually having an average diameter of 100 to 1000 nm.

2. A catalyst as claimed in Claim 1, wherein from 1 to 25 percent of the pore volume is in pores having individually an average diameter of from 100 to 1000 nm.

3. A catalyst as claimed in Claim 2 wherein from 2 to 25 percent of the pore volumes is in pores having individually an average diameter of from 100 to 1000 nm.

4. A catalyst as claimed in any preceding claim wherein from 55 to 90 percent of the pore volume is in pores individually having an average diameter of from 4 to 12 nm.

5. A catalyst as claimed in Claim 4 wherein from 60 to 90 percent of the pore volume is in pores individually having an average diameter of from 4 to 12 nm.

6. A catalyst as claimed in any preceding claim wherein said support contains less than 0.1 percent sodium as $Na_2O$ and less than 0.15 percent silicon as $SiO_2$.

7. A catalyst as claimed in any preceding claim wherein said support contains less than 0.1 percent sulfur, and less than 0.06 percent iron as $Fe_2O_3$.

8. A catalyst as claimed in any preceding claim wherein the copper metal is present at 3 to 13 percent of the weight of the support.

9. A catalyst as claimed in any preceding claim wherein the support has been prepared by decomposing a trialkyl alumina to alpha aluminium monohydrate and calcining the alpha aluminium monohydrate to gamma alumina.

10. A catalyst as claimed in any preceding claim wherein the support comprises up to 50% by weight of alpha alumina.

11. A catalyst as claimed in any preceding claim wherein the support also has thereon an activator metal comprising silver, platinum, palladium, manganese, cobalt, nickel, chromium, or molybdenum.

12. A catalyst as claimed in Claim 11 wherein the activator metal is present at less than 15 percent of the weight of the copper metal.

13. A process for the hydrogenation of an alkyne comprising reacting the alkyne with hydrogen in the presence of a catalyst characterised in that the catalyst is a catalyst as claimed in any one of Claims 1 to 12.

14. A process for the removal of alkynes from a hydrocarbon stream containing at least one diene which comprises contacting the hydrocarbon stream with a catalyst under selective hydrogenation conditions characterised in that the catalyst is as claimed in any one of Claims 1 to 12.

15. A process for the removal of alkynes from a hydrocarbon stream containing 1,3-butadiene comprising contacting the stream with a catalyst comprising of copper metal and one or more of the activator metals silver, platinum, palladium, manganese, cobalt, nickel, chromium and molybdenum dispersed on an alumina support characterised in that the catalyst is as claimed in Claim 11 or Claim 12.

**Patentansprüche**

1. Katalysator zur Hydrierung von Alkynen, umfassend einen Träger mit darauf fein verteiltem Kupfermetall, dadurch gekennzeichnet, daß der Träger ein poröses gamma-Aluminiumoxid ist, das gegebenenfalls alpha-Aluminiumoxid enthält und das eine spezifische Oberfläche von 68 bis 350 $m^2/g$ hat, 50 bis 90% des Porenvolumens in Poren vorliegt, die jeweils einen Durchschnittsdurchmesser von 4 bis 12 nm habnen und 0,5 bis 25% des Porenvolumens in Poren vorliegt, die jeweils einen Durchschnittsdurchmesser von 100 bis 1000 nm haben.

2. Katalysator nach Anspruch 1, bei dem 1 bis 25% des Porenvolumens in Poren vorliegt, die jeweils einen Durchschnittlichen Durchmesser von 100 bis 1000 nm aufweisen.

3. Katalysator nach Anspruch 2, bei dem 2 bis 25% des Porenvolumens in Poren vorliegt, die jeweils einen durchschnittlichen Durchmesser von 100 bis 1000 nm aufweisen.

4. Katalysator nach einem der vorhergehenden Ansprüche, bei dem 55 bis 90% des Porenvolumens in Poren vorliegt, die jeweils einen durchschnittlichen Durchmesser von 4 bis 12 nm aufweisen.

5. Katalysator nach Anspruch 4, bei dem 60 bis 90% des Porenvolumens in Poren vorliegt, die jeweils einen durchschnittlichen Durchmesser von 4 bis 12 nm aufweisen.

6. Katalysator nach einem der vorhergehenden Ansprüche, bei dem der Träger weniger als 0,1% Natrium als $Na_2O$ und weniger als 0,15% Silikon als $SiO_2$ enthält.

7. Katalysator nach einem der vorhergehenden Ansprüche, bei dem der Träger weniger als 0,01% Schwefel und weniger als 0,06% Eisen als $Fe_2O_3$ enthält.

8. Katalysator nach einem der vorhergehenden Ansprüche, bei dem das Kupfermetall zu 3 bis 13 Gew.-% des Trägers vorliegt.

9. Katalysator nach einem der vorhergehenden Ansprüche, bei dem der Träger durch Zersetzen eines Trialkylaluminas zum alpha-Aluminiummonohydrat und Kalzinieren des alpha-Aluminiummonohydrats zum gamma-Aluminiumoxid hergestellt wurde.

10

10. Katalysator nach einem der vorhergehenden Ansprüche, bei dem der Träger bis zu 50 Gew.-% alpha-Aluminiumoxid enthält.

11. Katalysator nach einem der vorhergehenden Ansprüche, bei dem der Träger auch ein Aktivatormetall, umfassend Silber, Platin, Palladium, Mangan, Kobalt, Nickel, Chrom oder Molybdän darauf enthält.

12. Katalysator nach Anspruch 11, bei dem das Aktivatormetall zu weniger als 15% des Gewichtes des Kupfermetalles vorliegt.

13. Verfahren zum Hydrieren eines Alkyns, umfassend das Umsetzen des Alkyns mit Wasserstoff in Gegenwart eines Katalysators, dadurch gekennzeichnet, daß der Katalysator ein Katalysator nach einem der Ansprüche 1 bis 12 ist.

14. Verfahren zum Entfernen von Alkynen aus einem Kohlenwasserstoffstrom, der zumindest ein Dien enthält, umfassend das Inkontaktbringen des Kohlenwasserstoffstromes mit einem Katalysator unter selektiven Hydrierungsbedingungen, dadurch gekennzeichnet, daß ein Katalysator nach einem der Ansprüche 1 bis 12 verwendet wird.

15. Verfahren zum Entfernen von Alkynen aus einem Kohlenwasserstoffstrom, der 1,3-Butadien enthält, umfassend das Inkontaktbringen des Stromes mit einem Katalysator, der Kupfermetall und ein oder mehrere der Aktivatormetalle Silber, Platin, Palladium, Mangan, Kobalt, Nickel, Chrom und Molybdän auf einem Aluminiumoxidträger dispergiert umfaßt, dadurch gekennzeichnet, daß ein Katalysator nach einem der Ansprüche 11 oder 12 verwendet wird.

**Revendications**

1. Catalyseur pour l'hydrogénation des alcynes, comprenant un support sur lequel il y a du métal cuivre finement divisé, caractérisé en ce que le support est une alumine-gamma, poreuse, contenant éventuellement de l'alumine-alpha et ayant une aire spécifique de 68 à 350 $m^2/g$, 50 à 90 pour cent du volume des pores se trouvant dans des pores ayant individuellement un diamètre moyen de 4 à 12 nm, et 0,5 à 25 pour cent du volume des pores se trouvant dans des pores ayant individuellement un diamètre moyen de 100 à 1000 nm.

2. Catalyseur selon la revendication 1, dans lequel de 1 à 25 pour cent du volume des pores se trouve dans des pores ayant individuellement un diamètre moyen de 100 à 1000 nm.

3. Catalyseur selon la revendication 2, dans lequel de 2 à 25 pour cent du volume des pores se trouve dans des pores ayant individuellement un diamètre moyen de 100 à 1000 nm.

4. Catalyseur selon l'une quelconque des revendications précédentes, dans lequel de 55 à 90 pour cent du volume des pores se trouve dans des pores ayant individuellement un diamètre moyen de 4 à 12 nm.

5. Catalyseur selon la revendication 4, dans lequel de 60 à 90 pour cent du volume des pores se trouve dans des pores ayant individuellement un diamètre moyen de 4 à 12 nm.

6. Catalyseur selon l'une quelconque des revendications précédentes, dans lequel ledit support contient moins de 0,1 pour cent de sodium sous forme de $Na_2O$ et moins de 0,15 pour cent de silicium sous forme de $SiO_2$.

7. Catalyseur selon l'une quelconque des revendications précédentes, dans lequel ledit support contient moins de 0,01 pour cent de soufre, et moins de 0,06 per cent de fer sous forme de $Fe_2O_3$.

8. Catalyseur selon l'une quelconque des revendications précédentes, dans lequel le métal cuivre est présent entre 3 et 13 pour cent du poids du support.

9. Catalyseur selon l'une quelconque des revendications précédentes, dans lequel on a préparé le support en décomposant un trialkoxy-aluminium en alumine alpha monohydratée et en calcinant l'alumine alpha monohydratée en alumine-gamma.

10. Catalyseur selon l'une quelconque des revendications précédentes, dans lequel le support comprend jusqu'à 50% en poids d'alumine-alpha.

11. Catalyseur selon l'une quelconque des revendications précédentes, dans lequel le support porte également sur lui un métal promoteur englobant l'argent, le platine, le palladium, le manganèse, le cobalt, le nickel, le chrome ou le molybdène.

12. Catalyseur selon la revendication 11, dans lequel le métal promoteur est présent à moins de 15 pour cent du poids du métal cuivre.

13. Procédé pour l'hydrogénation d'un alcyne, comprenant la réaction de l'alcyne avec l'hydrogène, en présence d'un catalyseur, caractérisé en ce que le catalyseur est un catalyseur selon l'une quelconque des revendications 1 à 12.

14. Procédé pour l'elimination des alcynes d'une courant d'hydrocarbures contenant au moins un diène, qui comprend la mise en contact du courant d'hydrocarbures avec un catalyseur dans des conditions sélectives d'hydrogénation, caractérisé en ce que le catalyseur est tel que revendiqué dans l'une quelconque des revendications 1 à 12.

15. Procédé pour l'elimination des alcynes d'un courant d'hydrocarbures contenant du 1,3-butadiène, qui comprend la mise en contact du courant avec un catalyseur comprenant le métal cuivre et un ou plusieurs métaux promoteurs, tels que l'argent, le platine, le palladium, le manganèse, le cobalt, le nickel, le chrome et le molybdène, dispersés sur un support d'alumine, caractérisé en ce que le catalyseur est tel que revendiqué dans la revendication 11 ou 12.

FIG. I

1

FIG. 2

FIG. 3

0 139 051

FIG. 4

4

FIG. 5

FIG. 6

FIG. 7

0 139 051

FIG. 8

0 139 051

FIG. 9

0 139 051

FIG. 10

FIG. 11

10

FIG. 12

FIG. 13

0 139 051

FIG. 14

FIG. 15

13

FIG. 16

14

FIG. 17

FIG. 18

0 139 051

FIG.19

FIG. 20

SAMPLE NUMBER, SAMPLE TREATMENT, AND $\dfrac{R_{X\text{-RAY}}}{R_{ESCA}}$

FIG.21